# EUROPÄISCHE PATENTSCHRIFT

(11) **EP 3 174 488 B1**
(45) Veröffentlichungstag und Bekanntmachung des Hinweises auf die Patenterteilung: **22.03.2023**
(21) Anmeldenummer: 15738272.2
(22) Anmeldetag: 09.07.2015
(51) Int. Cl.: A61B 90/50, A61G 12/00

(54) **BUCHSE, TRAGARM UND TRAGSYSTEM FÜR EINE MEDIZINTECHNISCHE STATIVVORRICHTUNG**
SOCKET, SUPPORTING ARM AND SUPPORT SYSTEM FOR A MEDICAL STAND DEVICE
DOUILLE, BRAS DE SUPPORT ET SYSTÈME DE SUPPORT POUR UN SOCLE MÉDICAL

(30) Priorität: 30.07.2014 EP 14002657
(43) Veröffentlichungstag der Anmeldung: 07.06.2017
(73) Patentinhaber: Ondal Medical Systems GmbH, 36088 Hünfeld (DE)
(72) Erfinder: OGINSKI, Stefan, 36041 Fulda (DE); BODE, Axel, 61206 Wöllstadt (DE); HÖSER, Markus, 36142 Tann (DE)
(74) Vertreter: Graf von Stosch, Andreas
(86) Internationale Anmeldenummer: PCT/EP2015/001397
(87) Internationale Veröffentlichungsnummer: WO 2016/015820

(56) Entgegenhaltungen:
- EP-A1- 2 060 844
- WO-A1-2010/072212
- WO-A2-2006/104566
- DE-A1-102012 112 712
- DE-B3-102004 054 866
- US-A1- 2008 086 150

## Beschreibung

Die vorliegende Erfindung betrifft eine Buchse für eine Stativvorrichtung zur Anordnung im Operationssaal, die eingerichtet ist, einen Tragarm oder eine medizintechnische Einrichtung an einer Spindel zu lagern. Die vorliegende Erfindung betrifft ferner einen an der Buchse befestigbaren Tragarm. Auch betrifft die vorliegende Erfindung ein Tragsystem mit wenigstens einer solchen Buchse und mit wenigstens einem solchen Tragarm. Die vorliegende Erfindung betrifft eine Stativvorrichtung mit einzelnen Merkmalen des Anspruchs 1 sowie deren Verwendung wie in Anspruch 15 beschrieben.

Relevanter Stand der Technik wird beispielsweise in den Dokumenten DE 10 2012 112712 A1, US 2008/086150 A1, DE 10 2004 054866 B3, WO 2010/072212 A1, EP 2 060 844 A1 und insbesondere WO 2006/104566 A2 offenbart.

Stative, insbesondere Deckenstative wie z.B. Deckenversorgungseinheiten, Monitorträger, oder so genannte Federarme oder Zentralachsen, weisen meist mehrere in Bezug auf eine Vertikalposition starr angeordnete oder höhenverstellbare Tragarme auf, mittels welchen eine daran befestigte medizintechnische Einrichtung bewegt und positioniert werden kann, z.B. im Operationssaal, insbesondere auch auf einer Intensivstation. An den Stativen sind häufig Versorgungseinheiten montiert, an welchen z.B. medizinisch-elektrische Endgeräte angeordnet sind, die z.B. während einer Operation mit den benötigten Medien versorgt werden. Die Träger/Tragarme definieren dabei einen Aktionsradius der medizintechnischen Einrichtung, in welchem die medizintechnische Einrichtung innerhalb des Operationssaals positioniert werden kann. Die Tragarme können daher auch als Ausleger bezeichnet werden. Die Tragarme können meist zumindest um wenigstens eine drehbare Verbindung, insbesondere ein Drehgelenk verdreht werden. Wahlweise sind die Tragarme auch höhenverstellbar und/oder um eine zumindest annähernd horizontal ausgerichtete Achse in der Höhe verschwenkbar angeordnet.

Bei solchen Stativen ist eine möglichst einfache Art der Montage und eine hohe Stabilität wünschenswert. Die einzelnen Tragarme des Stativs bilden dabei ein Tragsystem, welches häufig mehrere Gelenke oder Lager, insbesondere Drehgelenke umfasst. Ein jeweiliger Tragarm muss dann mit diesen Gelenken oder Lagern verbunden werden. Dies kann bekanntermaßen durch eine Schweißverbindung zwischen einem Tragarm und z.B. einer Buchse sichergestellt werden, insbesondere wenn die Komponenten aus Baustahl ausgeführt sind. Ein Verschweißen oder eine Schweißverbindung hat jedoch oftmals den Nachteil, dass Materialspannungen und möglicherweise auch Verzug im Tragsystem hervorgerufen wird. Insbesondere bei komplexeren Tragsystemen kann dies dazu führen, dass sich einzelne Komponenten nicht mehr exakt zueinander ausrichten lassen, oder dass die Gelenke/Lager nicht symmetrisch belastet werden. Das Tragsystem schwenkt dann möglicherweise aufgrund einwirkender Gravitationskräfte selbständig unvorhersehbar in eine bestimmte Richtung und wäre daher ohne eine Feststellbremse nicht verwendbar. Es kann sogar der Fall eintreten, dass die einzelnen Komponenten bei der Montage gar nicht erst aneinander montiert/gekuppelt werden können. Dies führt zu einer kostspieligen, zeitaufwändigen Montage, zu erforderlichen Korrekturmaßnahmen, oder zu Ausschuss und Mehrverbrauch. Um diesem Problem abzuhelfen, könnten die Lagerflächen in den Gelenken beispielsweise erst nach dem Herstellen der Schweißverbindung eingearbeitet oder überarbeitet werden, was jedoch sehr kostspielig ist.

Eine Schweißverbindung kann auch aus den folgenden Gründen nachteilig sein. Es müssen bestimmte, gut schweißbare Werkstoffe verwendet werden. Die Materialauswahl ist eingeschränkt. Insbesondere wird regelmäßig Baustahl bevorzugt, nicht zuletzt auch aus Kostengründen. Baustahl weist ein hohes Gewicht auf. Ein geschweißtes Tragsystem weist demnach meist auch ein hohes Eigengewicht auf, was auch die Montage erschwert und entsprechend dimensionierte, kostspielige Gelenke erfordert, insbesondere bei komplexeren Stativ-Systemen. Das gesamte System wird dann sehr träge und lässt sich nicht ohne beträchtlichen manuellen oder motorischen Kraftaufwand umpositionieren. Dies kann große Nachteile bei einer Operation haben, insbesondere wenn die Operation über einen langen Zeitraum von mehreren Stunden oder Tagen andauern muss. Baustahl muss zudem auch gegen Korrosion geschützt werden, und erfordert somit in vielen Fällen eine Beschichtung, insbesondere eine Pulverbeschichtung. Aber auch mit einer Beschichtung können Korrosionsschäden nicht ganz vermieden werden, insbesondere nicht in Hohlräumen oder Nuten für Kabel.

Ein Verschweißen bzw. eine Schweißverbindung erfordert zudem in vielen Fällen einen hohen logistischen (innerbetrieblichen) Aufwand, da beispielsweise die meist in unterschiedlichen örtlichen Bereichen eines Betriebs durchgeführten Schritte Vorfertigung, Schweißen, Nachbearbeitung, Qualitätskontrolle der Schweißnähte, Oberflächenbehandlung und Endmontage aufeinander abgestimmt werden müssen. Nicht zuletzt besteht beim automatisierten Schweißen das Risiko, dass ein Schweißroboter nicht exakt eingestellt ist, so dass eine Schweißnaht falsch positioniert wird und/oder nicht die erforderliche Tiefe aufweist. Diese Fehler sind bei einer Sichtprüfung nicht auf einfache Weise erkennbar.

Es ist eine Aufgabe der vorliegenden Erfindung, eine Vorrichtung oder Anordnung für ein Stativ, insbesondere Deckenstativ bereitzustellen, mittels welchem ein Stativ auf einfache Weise ausgelegt, hergestellt und/oder montiert werden kann, oder mittels welchem insbesondere die Ausrichtung oder Lage-Einstellung des Stativs erleichtert werden kann. Insbesondere besteht die Aufgabe darin, mindestens eine Komponente eines Tragsystems bereitzustellen, mittels welcher ein modulartiger Aufbau des Stativs auf einfache Weise erfolgen kann, insbesondere auch in Hinblick auf eine besonders exakte Ausrichtung. Die Aufgabe kann auch darin gesehen werden, ein Stativ so auszubilden, dass es auf einfache Weise für bestimmte Positionen oder Anordnungen im Operationssaal angepasst werden kann.

Diese Aufgabe wird gelöst durch eine Stativvorrichtung gemäß Anspruch 1. Hierdurch können mehrere Vorteile erzielt werden. Zum einen kann eine Materialwahl weitgehend frei erfolgen. Zum anderen wird ein modulartig aufgebautes Tragsystem bereitgestellt. Ferner kann eine Nachjustage auf einfache Weise erfolgen. Auch können unterschiedliche Varianten von Auslegern oder Tragarmen miteinander verbunden werden. Es kann z.B. ein bereits montierter Tragarm gegen einen anderen, längeren oder kürzeren Tragarm oder gegen einen Tragarm mit einer größeren Tragkraft ausgewechselt werden. Eine Stativvorrichtung mit einer solchen Buchse kann z.B. bei einer Restrukturierung oder Umfunktionierung eines Operationssaals auf einfache Weise angepasst werden. Nicht zuletzt kann die Montage an frei wählbaren Abschnitten der Wertschöpfungskette erfolgen.

Die jeweilige Systemkomponente wird thermisch nicht belastet. Es besteht kein Risiko eines Verzugs oder von Materialspannungen bei der jeweiligen Systemkomponente. Die Montage kann auf einfachere oder genauere Weise erfolgen. Eine verzugsbedingte Schiefstellung kann vermieden werden. Es kann ein Tragsystem bereitgestellt werden, bei welchem eine medizintechnische Einrichtung exakt ausgerichtet in einer vordefinierten Position gelagert werden kann, ohne dass das Tragsystem notwendigerweise mittels einer Feststellbremse in der Position verankert werden muss.

Dank der erfindungsgemäßen Art einer reversiblen Verbindung/Kupplung kann die Wahl des Werkstoffs der jeweiligen Systemkomponenten unabhängig von der Anforderung "schweißbar" erfolgen. Beispielsweise kann Aluminium problemlos verwendet werden. Nicht zuletzt deshalb kann die Buchse auch reversibel an einem z.B. stranggepressten Tragarmprofil befestigt/montiert werden. Es können auch besonders gewichtsoptimierende, insbesondere nichtmetallische Werkstoffe gewählt werden. Eine Beschichtung oder ein sonstiger Korrosionsschutz ist nicht erforderlich. Beispielsweise kann glas- oder kohlefaserverstärkter Kunststoff (GFK bzw. CFK) verwendet werden.

Hierdurch kann auch ermöglicht werden, einzelne Systemkomponenten modulartig miteinander in unterschiedlichen Anordnungen zu verbinden, so dass eine (jeweilige) Drehachse in einer spezifischen absoluten Raumposition anordenbar ist. Insbesondere kann der Abstand einzelner Drehachsen in horizontaler Richtung zueinander variiert werden, z.B. über die Länge des Tragarms oder eine Mehrzahl von aneinander montierten, insbesondere miteinander verschraubten Tragarmen.

Als "reversibel montierbar" ist dabei bevorzugt eine Anordnung zu verstehen, bei welcher die Komponenten aneinander fixiert werden können und auch wieder demontiert werden können oder in einer anderen Position aneinander montiert werden können.

Als Stativvorrichtung ist dabei bevorzugt eine Vorrichtung zum Halten, ortsfesten Anordnen und/oder Verlagern mindestens einer medizintechnische Einrichtung zu verstehen, die an einer Wand (in einem Wandlager) oder einer Raumdecke oder auch am Boden eines Operationssaals oder irgendeines anderen Raumes für medizinische Zwecke fest montiert oder positioniert werden kann, also z.B. ein Deckenstativ. Die Stativvorrichtung ist dann nicht vollkommen frei im Operationssaal verlagerbar, sondern kann nur in einem bestimmten Aktionsradius verlagert werden, insbesondere relativ zu einem an einer Raumdecke oder Wand des Operationssaals angeordneten Befestigungspunkt bzw. Montagepunkt. Die Stativvorrichtung kann als an einer Raumdecke montierte Deckenversorgungseinheit ausgebildet sein und eine oder mehrere Versorgungskonsolen aufweisen, welche an einem oder zwei Tragarmen gelagert und positionierbar ist. Die Stativvorrichtung kann auch als Monitorträger ausgebildet sein. Die Stativvorrichtung kann auch als so genannter, insbesondere an einer Wand montierter Federarm ausgebildet sein und z.B. eine Leuchte aufweisen. Die Stativvorrichtung kann auch als so genannte, insbesondere an einer Raumdecke montierte Zentralachse ausgebildet sein und eine Mehrzahl von Tragsystemen mit jeweils mindestens einem Träger aufweisen, an welchem z.B. ein Monitor oder eine Leuchte gelagert ist. Bevorzugt weist die Stativvorrichtung mindestens zwei Tragarme auf, an welchen jeweils mindestens eine Bremse der Bremseinrichtung vorgesehen sein kann. Beispielsweise können Feststellbremsen vorgesehen sein, welche dauerhaft und nur so leicht bremsen, dass ein Tragarm daran gehindert wird, selbstständig die Position zu verändern (Weglaufen, Wegschwenken). Für Montagezwecke kann eine solche Feststellbremse auch fester angezogen werden.

Als medizintechnische Einrichtung ist dabei bevorzugt eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Als Kupplungsteil ist dabei bevorzugt ein Teil der Buchse zu verstehen, mittels welchem der Tragarm oder Ausleger an einer Spindel oder einem anderen ortsfest angeordneten Verbindungsbauteil lagerbar ist, insbesondere drehbar oder auch höhenverstellbar. Der Kupplungsteil kann dabei z.B. zumindest einen Teil eines Drehlagers bilden, z.B. ringförmige Lagerflächen.

Als Verbindungsbauteil ist dabei bevorzugt ein Bauteil zu verstehen, mittels welchem einzelne Träger/Tragarme der Stativvorrichtung an einem ortsfesten Fixpunkt, insbesondere an einer Montagevorrichtung wie z.B. einem Deckenrohr unter einer Raumdecke, drehbar und/oder höhenverstellbar gelagert werden können. Bei einer Stativvorrichtung in Form einer so genannten Zentralachse kann das Verbindungsbauteil als zentral angeordnete, insbesondere rotationssymmetrische Spindel ausgebildet sein, an welcher mehrere Träger oder Tragarme gelagert sind.

Gemäß einem Ausführungsbeispiel ist der Montageabschnitt eingerichtet, die Buchse ohne Stoffschluss form- und/oder kraftschlüssig, bevorzugt zumindest im Wesentlichen kraftschlüssig, in einer vordefinierten Relativposition reversibel montierbar/demontierbar am Tragarm zu befestigen. Hierdurch kann ausgeschlossen werden, dass durch die Montage Positionsungenauigkeiten auftreten oder das Gesamtsystem (die Stativvorrichtung) nicht exakt ausgerichtet ist. Beispielsweise kann die Befestigung mittels einer im Wesentlichen kraftschlüssig wirkenden Schraubverbindung erfolgen.

Gemäß einem Ausführungsbeispiel weist der Montageabschnitt wenigstens eine Durchführung zur Anordnung eines Befestigungsmittels auf. Hierdurch kann die Montage vereinfacht werden, und eine Kraftübertragung kann an vordefinierten Befestigungspunkten erfolgen. Bevorzugt erstreckt sich die Durchführung in radialer Richtung, insbesondere zumindest annähernd orthogonal zur Drehachse. Hierdurch können die Befestigungsmittel im Wesentlichen auf Zug beansprucht werden oder auch mit einer vergleichsweise kleinen Vorspannung montiert werden. Bevorzugt weist der Montageabschnitt wenigstens zwei in einem bevorzugt maximal großen Abstand zueinander angeordnete Durchführungen auf. Hierdurch können Drehmomente auf effektive Weise übertragen werden.

Die Durchführung kann z.B. als Bohrung oder Ausfräsung ausgebildet sein. Bevorzugt sind mehrere Durchführungen, insbesondere vier oder mehr Durchführungen, umlaufend entlang einer Kontur entsprechend einer Außenkontur des Tragarms angeordnet. Hierdurch können hohe Stützkräfte oder Momente übertragen werden.

Gemäß der Erfindung weist der Montageabschnitt wenigstens eine zur Drehachse weisende Anlagefläche auf, an welcher ein Befestigungsmittel zur Übertragung einer nach radial außen in radialer Richtung im Wesentlichen orthogonal zur Drehachse wirkenden Normalkraft zur Anlage bringbar ist. Hierdurch kann auch eine allein oder zumindest im Wesentlichen durch Kraftschluss wirkende Schnittstelle zwischen der Buchse und dem Tragarm bereitgestellt werden.

Gemäß einem Ausführungsbeispiel ist der Montageabschnitt eingerichtet, die Buchse im Wesentlichen mittels Kraftschluss durch Haftreibung am Tragarm zu befestigen, und/oder mittels durch Kraftschluss herbeigeführtem Formschluss. Hierdurch kann eine Schnittstelle bereitgestellt werden, insbesondere eine ebene Fläche, welche auf einfache und kostengünstige Weise hergestellt werden kann. Eine Haftreibung hervorrufende Normalkraft kann dabei z.B. durch Befestigungsmittel in Form von Schrauben zwischen der Buchse und dem Tragarm übertragen werden. Wahlweise kann auch Formschluss erfolgen, z.B. mittels irgendwelcher Absätze, Ränder oder Kanten am Montageabschnitt. Bevorzugt erfolgt Kraftschluss in Verbindung mit Formschluss. Der Montageabschnitt kann auch durch Absätze, Rastmittel oder Hinterschneidungen eine Verbindung bereitstellen, bei welcher zunächst eine Kraft aufgebracht werden muss, um eine formschlüssige Kupplung bereitzustellen. Formschluss kann dabei die Montage erleichtern oder auch die Sicherheit oder Robustheit der Kupplung erhöhen.

Gemäß einem Ausführungsbeispiel weist der Montageabschnitt eine nach radial außen weisende Stirnseite auf, welche geometrisch korrespondierend zu einer Stirnseite des Tragarms ausgebildet ist, insbesondere zumindest annähernd in einer Ebene angeordnet ist. Hierdurch kann die Montage auf einfache Weise an einer Seite erfolgen, an welcher Kräfte und Momente effektiv und über eine vergleichsweise große Fläche übertragen werden können. Bevorzugt weist die Stirnseite eine ebene Stirnfläche auf, welche eine Schnittstelle zu einer geometrisch korrespondierend ausgebildeten Stirnfläche (einer Montageschnittstelle) des Tragarms bildet. Dies ermöglicht eine Anordnung auf Stoß, wobei die Komponenten auf einfache Weise mit hoher Passgenauigkeit hergestellt werden können. Es müssen nur große oder überhaupt keine Maßtoleranzen beachtet werden. Es hat sich jedoch gezeigt, dass es vorteilhaft ist, die aneinander anliegenden Komponenten in Hinblick auf bestimmte Rechtwinkligkeitstoleranzen auszulegen, insbesondere um eine besonders exakte Ausrichtung der Tragarme sicherstellen zu können.

Bevorzugt ist der Montageabschnitt durch wenigstens einen zumindest annähernd parallel zur Drehachse ausgerichteten Flächenabschnitt der Stirnseite gebildet. Hierdurch kann ein einfacher Aufbau, eine einfache Montage und/oder eine symmetrische Krafteinleitung sichergestellt werden. Gemäß einer bevorzugten Variante ist die Stirnseite durch eine ebene Stirnfläche gebildet. Dies ermöglicht ein Nachbearbeiten des Montageabschnitts auf einfache Weise. Auch kann dabei eine Ausrichtung des Stativs auf einfache oder besonders exakte Weise nachjustiert werden.

Gemäß einer vorteilhaften Variante ist der Flächenabschnitt der Stirnseite leicht geneigt (ca. 0.2° bis 0.5°, insbesondere 0.3°), so dass ein Tragarm im unbelasteten Zustand leicht nach oben ausgerichtet ist und unter Belastung (Gravitationskraft) nicht nach unten biegt oder durchhängt. Mit anderen Worten ermöglicht der Flächenabschnitt eine Ausrichtung des Tragarms auf sehr exakte Weise. Dabei kann der Flächenabschnitt auf vergleichsweise einfache Weise nachbearbeitet werden.

Der Flächenabschnitt der Stirnseite ist in Bezug auf den Querschnitt der Buchse in einer Ebene parallel zur Drehachse ("Radialquerschnitt") bevorzugt maximal groß, insbesondere in Hinblick auf eine gute Stabilität und/oder um große Biegemomente übertragen zu können. Weiter bevorzugt weist der Radialquerschnitt in Richtung der Drehachse wenigstens zumindest annähernd dieselbe (axiale) Erstreckung auf wie der Kupplungsteil, insbesondere aus Stabilitätsgründen. Gemäß einer Variante kann der Radialquerschnitt auch größer sein als die axiale Erstreckung des Kupplungsteils, insbesondere falls dies eine verbesserte Stabilität sicherstellen kann.

Gemäß einem Ausführungsbeispiel weist der Montageabschnitt eine Sicherung zum Abstützen des Tragarms auf, insbesondere eine Sicherung eingerichtet zum Halten des Tragarms unabhängig von zusätzlichen Befestigungsmitteln. Diese Sicherung erleichtert nicht zuletzt die Montage, insbesondere indem der Tragarm an/auf der Sicherung gelagert wird, bis der Tragarm vollständig befestigt ist. Die Sicherung kann einen hohen Sicherheitsfaktor sicherstellen und zusätzlich zu Befestigungsmitteln in der Art einer redundanten Sicherung wirken.

Gemäß einem Ausführungsbeispiel steht die Sicherung in radialer Richtung hervor, wobei die Sicherung bevorzugt ein Querschnittsprofil aufweist, welches geometrisch korrespondierend zum Querschnittsprofil des Tragarms ausgebildet ist. Bevorzugt ist das Querschnittsprofil der Sicherung kleiner als das Querschnittsprofil des Tragarms, so dass die Sicherung in das Querschnittsprofil des Tragarms eingreifen kann. Die Sicherung kann dabei in der Art einer Stecker-Buchse-Verbindung ausgebildet sein, wobei der Tragarm die Seite der Buchse bildet, in welche die Sicherung eingreift. Die Sicherung, ist bevorzugt als länglicher Profilabschnitt ausgebildet, der radial von der Buchse abragt.

Vorteilhaft weist der Tragarm einen Innenraum zur drehfesten Aufnahme der als Längsprofil ausgebildeten Sicherung der Buchse auf, wobei an einer Seite des Innenraums ein Lagerungsabschnitt ausgebildet ist, der zum Innenraum hin eine Lagerfläche aufweist, deren Kontur mit einer Auflagekontur der Sicherung der Buchse korrespondiert, so dass im eingesteckten Zustand der Sicherung in den Innenraum die Lagerfläche am Tragarm sich in, insbesondere formschlüssiger, Anlage mit der korrespondierenden Auflagekontur der Sicherung an der Buchse befindet.

Die Sicherung ist zur Montage des Tragarms an der Buchse also in den axial verlaufenden Innenraum des Tragarms einführbar. Die Auflagekontur der Sicherung bzw. des länglichen Profilabschnitts kommt dabei in Anlage an eine korrespondierende Lagerfläche am Lagerungsabschnitt des Tragarms.

Gemäß einem Ausführungsbeispiel weist die Sicherung einen bevorzugt konkav nach innen und unten gewölbten Auflageabschnitt auf, welcher eingerichtet ist, den Tragarm zu lagern und in vertikaler Richtung abzustützen, wobei sich der Auflageabschnitt bevorzugt zumindest annähernd in radialer Richtung orthogonal zur Drehachse erstreckt. Bevorzugt weist der Auflageabschnitt eine Kontur auf, welche einer entsprechend (insbesondere konvex) gewölbten Innenkontur des Tragarms entspricht.

Gemäß einem Ausführungsbeispiel weist der Montageabschnitt wenigstens einen formschlüssigen Sicherungsabschnitt gegen Vertikalbewegung und/oder Verdrehen oder gegen Axialbewegung und/oder Verdrehen auf. Dies verbessert die Belastbarkeit des Montageabschnitts und kann die Montage erleichtern.

Gemäß einem Ausführungsbeispiel weist der Montageabschnitt eine Aussparung für Leitungen oder Kabel auf, insbesondere in radialer Richtung. Dies ermöglicht ein Verlegen von Kabeln oder Leitungen unabhängig davon, in welchem Zusammenbau-Zustand das System vorliegt. Bevorzugt ist die Aussparung für Kabel oder Leitungen oberhalb von der Sicherung angeordnet.

Gemäß einem Ausführungsbeispiel ist die Buchse gabelförmig und weist wenigstens zwei Ringabschnitte auf, welche den Kupplungsteil bilden, insbesondere zwei Ringabschnitte mit Lagersitzen für ein Drehlager. Hierdurch kann eine stabile, robuste Konstruktion bereitgestellt werden, welche wenig Materialeinsatz erfordert und Kräfte und Momente an wirkungsvollen Punkten übertragen kann.

Gemäß einem Ausführungsbeispiel ist die Buchse ein Gussteil, insbesondere aus Aluminium oder einer Aluminiumlegierung. Hierdurch kann die Buchse funktionsoptimiert und materialsparend ausgestaltet werden. Bei einem Gussteil lassen sich zudem Öffnungen oder Ausprägungen, die zum Befestigen weiterer Komponenten dienen können, kostengünstig mitgießen. Würde die Buchse beispielsweise als Schweißkonstruktion aus mehreren Platten hergestellt, müssten zusätzliche Anbauteile für die Aufnahme von z.B. Bremsschrauben, Drehwinkel-Anschläge, Ösen für die Verschraubung der Verkleidung, usw. angebracht werden.

Bei Aluminium (bzw. einer Alu-Legierung) als Werkstoff für die Buchse kann auch an der gegossenen Buchse möglicherweise erforderliche Nacharbeit reduziert werden oder auf einfachere Weise (geringerer Material- oder Energieeinsatz) ausgeführt werden.

Die zuvor genannte Aufgabe wird auch gelöst durch einen Tragarm für eine Stativvorrichtung zur Anordnung im Operationssaal, mit wenigstens einer Schnittstelle, an welcher der Tragarm mit einer Buchse der Stativvorrichtung verbindbar ist; wobei die Schnittstelle eine Montageschnittstelle ist, mittels welcher der Tragarm an der Buchse reversibel montierbar ist. Hierdurch ergeben sich zumindest teilweise die zuvor in Verbindung mit der Buchse genannten Vorteile. Der Tragarm kann dabei ohne Stoffschluss form- und/oder kraftschlüssig in einer vordefinierten Relativposition reversibel montierbar sein. An der Montageschnittstelle kann wenigstens eine Aufnahme zum Befestigen der Buchse angeordnet sein, insbesondere mittels wenigstens einem Befestigungsmittel. Bevorzugt ist die wenigstens eine Aufnahme in einem radial außenliegenden Abschnitt eines Querschnittsprofils des Tragarms angeordnet. Dies ermöglicht eine stabile Verbindung oder das Übertragen von hohen Kräften.

Bevorzugt weist der Tragarm an gegenüberliegenden Enden zwei zumindest annähernd identisch ausgebildete Montageschnittstellen auf. Hierdurch kann der Tragarm unabhängig von dessen Ausrichtung montiert werden. Auch können mehrere Tragarme auf einfache Weise aneinander montiert werden, insbesondere ohne dass ein Adapter erforderlich ist.

Gemäß einer Variante sind das vordere und hintere Ende des Tragarms voneinander unterschiedlich ausgebildet, indem am vorderen Ende seitens der medizintechnischen Einrichtung auf eine formschlüssige Sicherung verzichtet wird. Es hat sich gezeigt, dass aufgrund des Hebelarms die auf den vorderen Montageabschnitt des Tragarms ausgeübte Belastung deutlich geringer ist, so dass auf eine Sicherung verzichtet werden kann.

Bevorzugt ist das Querschnittsprofil des Tragarms zu einer Seite hin offen, insbesondere nach oben oder unten. Dies ermöglicht ein Verlegen eines Kabels durch seitliches Einlegen des Kabels. Bei einer nicht stoffschlüssigen Verbindung zur Buchse kann ein solcher Kabelkanal dabei auch bis zur Buchse vorgesehen werden und genutzt werden. Bei einer Schweißverbindung hingegen ist dies möglicherweise nicht möglich, insbesondere falls eine Schweißnaht aus Festigkeitsgründen umlaufend vorgesehen sein muss.

Bevorzugt weist der Tragarm ein rechteckiges, zu wenigstens einer Seite offenes Querschnittsprofil mit einem oberseitigen oder unterseitigen Kabelkanal auf. Bevorzugt ist der Kabelkanal in einem montierten Zustand des Tragarms nach oben oder unten hin offen, wobei der Tragarm von einer Verkleidung umgeben sein kann, hinter welcher ein Kabel gelagert werden kann. Bevorzugt ist der Tragarm durch ein Strangpressprofil gebildet.

Bevorzugt ist der Tragarm aus Aluminium oder einer Aluminiumlegierung gebildet. Bevorzugt ist der Tragarm aus einem Strangpressprofil gebildet. Bevorzugt weist der Tragarm vorgepresste Aufnahmen (insbesondere Schraubkanäle) auf, welche geometrisch korrespondierend zu den Befestigungsmitteln ausgebildet sind. Diese Art Aufnahmen erlaubt es auch, das Presswerkzeug besonders stabil herstellen, so dass mit höheren Pressgeschwindigkeiten gearbeitet werden kann. Das entsprechende Presswerkzeug kann auch kostengünstig ausgeführt werden. Bevorzugt weisen die Aufnahmen Innengewinde auf.

Bevorzugt weist der Tragarm einen Lagerungsabschnitt auf, welcher eingerichtet ist, an/auf einer Sicherung (insbesondere auf einem Auflageabschnitt) der Buchse gelagert zu werden. Der Lagerungsabschnitt ist bevorzugt konvex nach innen und unten gewölbt und an der Oberseite des Tragarms vorgesehen. Der Lagerungsabschnitt weist bevorzugt eine nach innen weisende Lagerfläche auf. Gemäß einer Variante bildet der Lagerungsabschnitt dabei auch gleichzeitig eine Kavität zur Aufnahme von Kabeln, insbesondere einen von oben zugänglichen Kabelkanal. Diese Ausgestaltung liefert zum einen eine hohe Stabilität, zum anderen eine gute Zugänglichkeit.

Der Lagerungsabschnitt ist eingerichtet, den Tragarm zu lagern und in vertikaler Richtung abzustützen, wobei sich der Lagerungsabschnitt bevorzugt zumindest annähernd in Längsrichtung des Tragarms erstreckt. Auf diese Weise kann der Tragarm in radialer Richtung auf die Buchse gesteckt werden. Bevorzugt ist das Querschnittsprofil des Tragarms durch eine umlaufende Wandung gebildet, wobei die wenigstens eine Aufnahme bevorzugt jeweils in einer Ecke der Wandung ausgebildet ist. Dabei kann eine Innenfläche der Wandung die Aufnahme bilden. Beispielsweise kann ein Innengewinde an der Innenfläche der Wandung ausgebildet sein. Die umlaufende Wandung ist bevorzugt an einer Seite des Tragarms, insbesondere an der Oberseite, konkav nach innen gewölbt, so dass sich im Querschnitt ein U-Profil ergibt. Dieses U-Profil kann einen formbeständigen Lagerungsabschnitt bilden.

Die zuvor genannte Aufgabe wird auch gelöst durch ein Tragsystem für eine Stativvorrichtung zur Anordnung im Operationssaal, umfassend wenigstens eine erfindungsgemäße Buchse und wenigstens einen erfindungsgemäßen Tragarm, welcher reversibel am Montageabschnitt der Buchse montiert oder montierbar ist. Das Tragsystem besteht dabei aus wenigstens zwei reversibel aneinander in vordefinierter Relativposition zueinander montierbaren Komponenten. Bevorzugt bilden zwei Buchsen und ein Tragarm jeweils einen bevorzugt drehlagerbaren Ausleger bestehend aus wenigstens drei reversibel montierbaren Komponenten.

Bei dem Tragsystem können die einzelnen Komponenten z.B. jeweils als Gussteil oder Strangpressprofil ausgebildet sein. Die Materialauswahl kann unabhängig von den anderen Komponenten erfolgen.

Gemäß einem Ausführungsbeispiel umfasst das Tragsystem Befestigungsmittel, bevorzugt in Form von Schrauben, welche an der Montageschnittstelle zwischen der Buchse und dem Tragarm montiert oder montierbar sind. Hierdurch kann die Montage z.B. mit einem vorgegebenen Drehmoment der Schrauben erfolgen, was die Sicherheit erhöhen kann und die Verbindung durabel gestalten kann. Mit Befestigungsmitteln in Form von Schrauben kann z.B. auf einfache Weise dokumentiert werden, mit welcher Vorspannung die Komponenten aneinander gekuppelt sind, insbesondere mit welchem Drehmoment die Befestigungsmittel befestigt worden sind. Die Befestigungsmittel können direkt im Zuge der Endmontage montiert werden, so dass der gesamte Herstellungs- oder Montageprozess schlank/effizient ist und Zeit und Kosten gespart werden können. Der Tragarm ist also über wenigstens ein, vorzugsweise vier Befestigungsmittel, die z. B. als Schrauben ausgebildet sind, an der Buchse zu fixieren, die die Sicherung an einer Ecke, bevorzugt vier Ecken flankieren, so dass eine sowohl drehfeste als auch in axialer Richtung des Tragarms bzw. in radialer Richtung der Buchse zugfeste Verbindung zwischen dem Tragarm und der Buchse erreicht wird.

Gemäß einer Variante werden Befestigungsmittel in der Art einer Steckverbindung vorgesehen, insbesondere über eine größere Länge. Die Steckverbindung kann z.B. durch zusätzliche seitlich eingebrachte Stifte gesichert werden, sofern gewünscht.

Die Befestigungsmittel können in radialer Richtung zumindest annähernd orthogonal zur Drehachse montiert oder montierbar sein, so dass sie im Wesentlichen auf Zug belastbar sind. Diese Anordnung liefert Vorteile hinsichtlich des Kraftflusses, insbesondere auch in Verbindung mit einer im Wesentlichen ebenen und parallel zur Drehachse ausgerichteten Montagefläche. Die Befestigungsmittel greifen bevorzugt formschlüssig in den Tragarm ein.

Dies ermöglicht einen einfachen Aufbau und eine einfache Montage. Die Befestigungsmittel müssen beispielsweise lediglich durch in die Buchse eingebrachte Durchführungen/Bohrungen gesteckt werden.

Gemäß einem Ausführungsbeispiel umfasst das Tragsystem eine weitere Buchse, welche reversibel an einer weiteren Montageschnittstelle des Tragarms montiert oder montierbar ist. Hierdurch kann mit nur drei Komponenten, die reversibel aneinander montierbar sind, eine vordefinierbare Anordnung bereitgestellt werden, beispielsweise von zwei Drehachsen.

Gemäß einem Ausführungsbeispiel umfasst das Tragsystem wenigstens zwei Buchsen, welche an einer jeweiligen Montageschnittstelle am Tragarm montiert oder montierbar sind, wobei die Montageschnittstellen jeweils an einem freien Ende des Tragarms angeordnet sind. Bevorzugt sind die Montageschnittstellen identisch ausgebildet. Hierbei kann die jeweilige Montageschnittstelle durch dasselbe Profil gebildet werden, insbesondere durch ein Stranggussprofil. Bei dieser Ausgestaltung kann ein Ausleger/Tragarm auch durch einen weiteren Tragarm verlängert werden. Bevorzugt weisen die Montageschnittstellen jeweils fluchtend zur anderen Montageschnittstelle angeordnete Aufnahmen für Befestigungsmittel auf. Hierdurch kann der Tragarm auf zweckdienliche Weise in Form eines Profils verwendet werden, an welchem die Kräfte in denselben Querschnittsbereichen angreifen und weitergeleitet werden können. Dies bevorzugt eine symmetrische Kraftverteilung und Kraftweiterleitung. Gemäß einer Variante definieren die Buchsen jeweils eine Drehachse. Bei zwei an einem jeweiligen Ende des Tragarms mittels der Buchsen bereitgestellten Drehachsen sind diese bevorzugt zumindest annähernd parallel zueinander ausgerichtet. Bei dieser Anordnung kann eine symmetrische Krafteinleitung und Kraftweiterleitung sichergestellt werden. Das Tragsystem kann sich hierdurch selbst stabilisieren.

Gemäß einem Ausführungsbeispiel umfasst ein Tragsystem für eine Stativvorrichtung zur Anordnung im Operationssaal wenigstens eine Buchse mit einem Kupplungsteil, mittels welchem die Buchse um eine Drehachse an einer Spindel der Stativvorrichtung lagerbar ist, und mit einem Montageabschnitt, mittels welchem die Buchse an einen Tragarm der Stativvorrichtung reversibel montierbar ist, und umfasst ferner den Tragarm oder wenigstens einen weiteren Tragarm mit einer entsprechenden Montageschnittstelle, wobei der Montageabschnitt und die Montageschnittstelle eingerichtet sind, die Buchse und den Tragarm ohne Stoffschluss form- und/oder kraftschlüssig in einer vordefinierten Relativposition reversibel montierbar/demontierbar aneinander zu befestigen, wobei der Montageabschnitt wenigstens eine Durchführung zur Anordnung eines Befestigungsmittels aufweist, wobei der Montageabschnitt wenigstens eine zur Drehachse weisende Anlagefläche aufweist, an welcher ein Befestigungsmittel zur Übertragung einer nach radial außen in radialer Richtung im Wesentlichen orthogonal zur Drehachse wirkenden Normalkraft zur Anlage bringbar ist, wobei der Montageabschnitt eine nach radial außen weisende Stirnseite aufweist, welche geometrisch korrespondierend zu einer Stirnseite des Tragarms ausgebildet ist, und wobei die Buchse eine Sicherung zum Halten des Tragarms aufweist. Der Montageabschnitt weist insbesondere eine ebene Stirnfläche auf, welche eine Schnittstelle zu einer geometrisch korrespondierend ausgebildeten Stirnfläche der Montageschnittstelle bildet. Hierdurch können eine Vielzahl der zuvor genannten Vorteile in Verbindung miteinander realisiert werden.

Die zuvor genannte Aufgabe wird auch gelöst durch eine Stativvorrichtung zur Anordnung im Operationssaal und zum Positionieren/Halten einer medizintechnischen Einrichtung im Operationssaal, umfassend wenigstens eine erfindungsgemäße Buchse und wenigstens einen erfindungsgemäßen Tragarm oder umfassend ein erfindungsgemäßes Tragsystem, wobei die Stativvorrichtung ein Verbindungsbauteil in Form einer Spindel aufweist, an welcher der Tragarm mittels der Buchse reversibel montiert oder montierbar ist.

Die vorliegende Erfindung betrifft auch die Verwendung einer Stativvorrichtung im Operationssaal zum Positionieren/Halten einer medizintechnischen Einrichtung, umfassend wenigstens eine Buchse und wenigstens einen Tragarm, wobei die Stativvorrichtung ein Verbindungsbauteil (insbesondere in Form einer Spindel) aufweist, an welchem der Tragarm mittels der Buchse reversibel montiert ist, wobei die Buchse einen Montageabschnitt aufweist, wobei der Tragarm eine Montageschnittstelle aufweist, und wobei der Montageabschnitt und die Montageschnittstelle geometrisch korrespondierend zueinander ausgebildet sind. Hierbei kann der modulartige Aufbau zum Einstellen oder Nachjustieren der Vorrichtung genutzt werden. Mit anderen Worten: Die zuvor genannte Aufgabe wird auch gelöst durch eine Verwendung einer Stativvorrichtung im Operationssaal zum Positionieren/Halten einer medizintechnischen Einrichtung, umfassend wenigstens eine Buchse und wenigstens einen Tragarm, wobei die Buchse einen Montageabschnitt aufweist, mittels welchem die Buchse reversibel an eine Montageschnittstelle des Tragarms montiert ist.

In den nachfolgenden Zeichnungsfiguren wird die Erfindung anhand eines Ausführungsbeispiels näher erläutert. Es zeigen:
- Figuren 1A, 1B, 1C: jeweils in perspektivischer Ansicht eine Buchse gemäß einem Ausführungsbeispiel der Erfindung;
- Figuren 2A, 2B, 2C: jeweils in einer Seitenansicht bzw. in einer Draufsicht von oben oder unten die in den Figuren 1A, 1B, 1C gezeigte Buchse;
- Figuren 3A, 3B, 3C, 3D: jeweils in perspektivischer Ansicht bzw. in einer Vorderansicht einen Tragarm für eine Buchse oder eine Stativvorrichtung gemäß einem Ausführungsbeispiel der Erfindung;
- Figuren 4A, 4B: jeweils in perspektivischer Ansicht eine weitere Buchse gemäß einem Ausführungsbeispiel der Erfindung;
- Figur 5: in perspektivischer Ansicht ein Tragsystem oder einen Teil eines Tragsystems, insbesondere einen Ausleger mit drei Komponenten, gemäß einem Ausführungsbeispiel der Erfindung; und
- Figur 6: in schematischer Darstellung eine Stativvorrichtung umfassend eine Mehrzahl von Buchsen und Tragarmen gemäß einem Ausführungsbeispiel der Erfindung.

Im Zusammenhang mit der Beschreibung der folgenden Figuren wird bei Bezugszeichen, falls sie in einzelnen Figuren nicht explizit erläutert werden, auf die weiteren Figuren verwiesen.

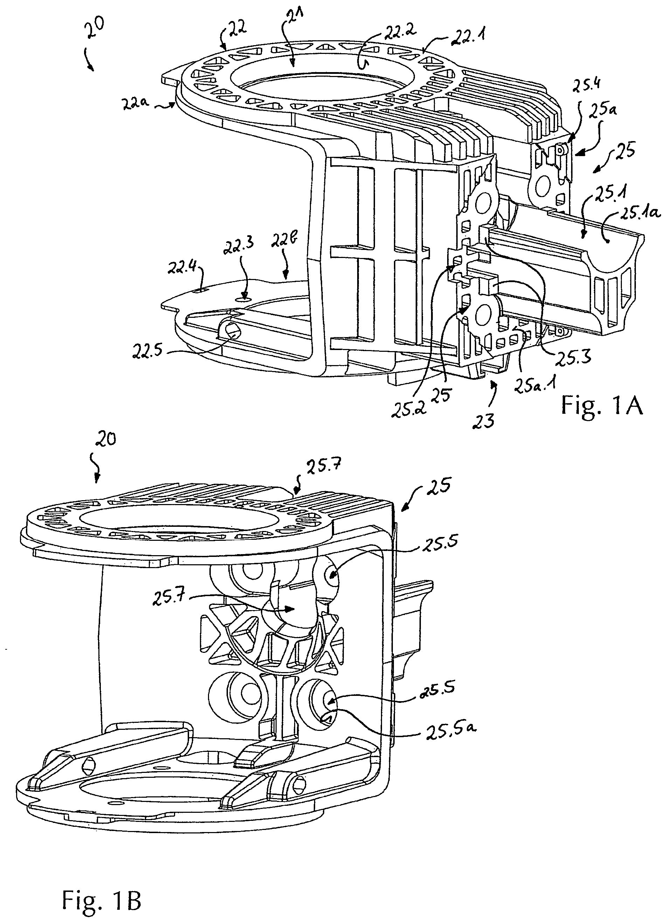

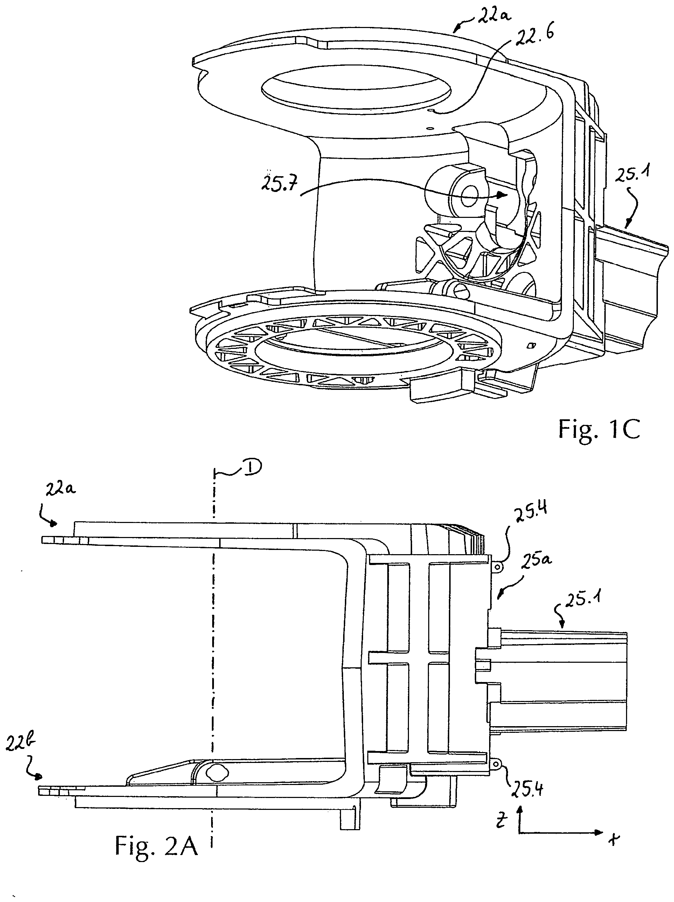

In den Figuren 1A, 1B, 1C ist eine Buchse 20 gezeigt, welche eingerichtet ist, an einer Spindel (nicht dargestellt) drehbar gelagert zu sein. Die Buchse weist einen Kupplungsteil 21 und einen Montageabschnitt 25 auf, wobei der Kupplungsteil 21 im Wesentlichen durch zwei Ringabschnitte 22, 22a, 22b gebildet ist. Am Montageabschnitt 25 kann ein Tragarm (nicht dargestellt) befestigt werden.

In der Figur 1A ist die Buchse 20 von der Seite des Montageabschnitts 25 gezeigt, wobei eine Stirnseite 25a sichtbar ist. Die Stirnseite 25a weist eine zumindest abschnittsweise ebene Stirnfläche 25a.1 auf. An der Stirnfläche 25a.1 kann ein Tragarm zur Anlage gebracht werden, und es können Normalkräfte übertragen werden, insbesondere im Kraftschluss durch Haftreibung sicherzustellen. An der Stirnseite 25a sind ein formschlüssiger Sicherungsabschnitt 25.2 gegen Vertikalbewegung und/oder Verdrehen und ein formschlüssiger Sicherungsabschnitt 25.3 gegen Axialbewegung und/oder Verdrehen ausgebildet. Ferner ist eine Schrauböse 25.4 zum Anschrauben einer Verkleidung angedeutet.

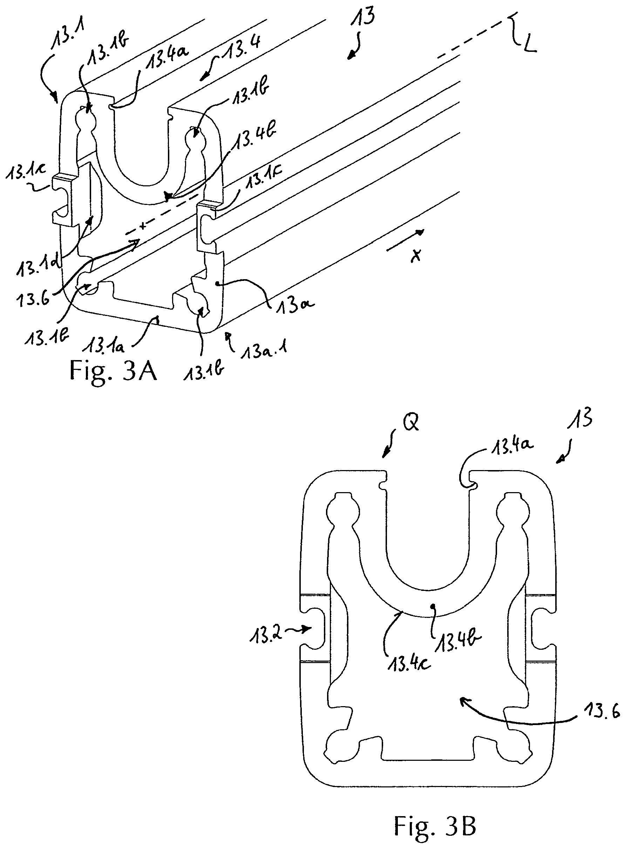

Der Sicherungsabschnitt 25.2 ist bevorzugt eine Ausfräsung bzw. eine Ausnehmung oder Vertiefung und weist eine obere und eine untere Fläche auf. Zwischen diesen beiden Flächen kann ein Zapfen 13.1c des Tragarms 13 (siehe Figur 3A) in Position gehalten werden. Eine oder mehrere Sicherungsabschnitte/Ausfräsungen 25.2 können zusammen große Torsionsmomente aufnehmen und den Tragarm gegen Verdrehen sichern, insbesondere bei großen dynamischen Impulsen oder Momenten, wenn der Tragarm ruckartig an einem oberen oder unteren Anschlag anschlägt. Ein Spalt zwischen den Sicherungsabschnitten 25.2 und dem Tragarm ist bevorzugt klein, ca. 0.1 mm.

Die Sicherungsabschnitte 25.3 liegen bevorzugt nicht so eng am Tragarm an. Dadurch übertragen sie auch kaum oder gar keine Torsionsmomente. Die Sicherungsabschnitte 25.3 sind eingerichtet, den Ausleger horizontal zu positionieren, insbesondere um Befestigungselemente/Schrauben auf einfache Weise montieren zu können.

Der Montageabschnitt 25 weist eine Sicherung 25.1 für den Tragarm auf, an/auf welcher der Tragarm abgestützt werden kann. Die Sicherung 25.1, ist bevorzugt als länglicher Profilabschnitt ausgebildet, der radial von der Buchse 20 abragt. Die Sicherung 25.1 weist einen Auflageabschnitt, eine Auflagefläche und/oder eine Auflagekontur 25.1a auf, auf welchem/welcher ein entsprechender Abschnitt des Tragarms lagerbar ist. Die Sicherung 25.1 ist zur Montage des Tragarms 13 an der Buchse 20 in einen axial verlaufenden Innenraum 13.6 des Tragarms 13 einführbar (vergleiche Figuren 1A, 3A und 5). Die Auflagekontur 25.1a kommt dabei in Anlage an eine korrespondierende Lagerfläche 13.4c am Lagerungsabschnitt 13.4b des Tragarms 13 (siehe Fig. 3A und 3B).

Der obere Ringabschnitt 22a des Kupplungsteils 21 weist eine Stirnfläche 22.1 auf, an welcher eine weitere Komponenten, beispielsweise eine weitere Buchse angeordnet werden kann. Jeder Ringabschnitt weist eine nach innen weisende Mantelfläche (Lagersitz) 22.2 auf, an welcher die Buchse 22 um die Spindel lagerbar ist.

Der untere Ringabschnitt 22b des Kupplungsteils 21 weist mehrere Ausnehmungen 22.3 zum Einrasten eines Schleifrings sowie mehrere Ausnehmungen 22.4, insbesondere Durchgangsbohrungen, zum Einrasten einer Abdeckkappe auf. Ferner sind eine oder mehrere Gewindebohrungen 22.5 für Bremsschrauben vorgesehen.

An der Unterseite der Buchse 20 ist ferner eine Aufnahme 23 für einen Riegel zum Einstellen einer Anschlagsposition bzw. Drehposition vorgesehen.

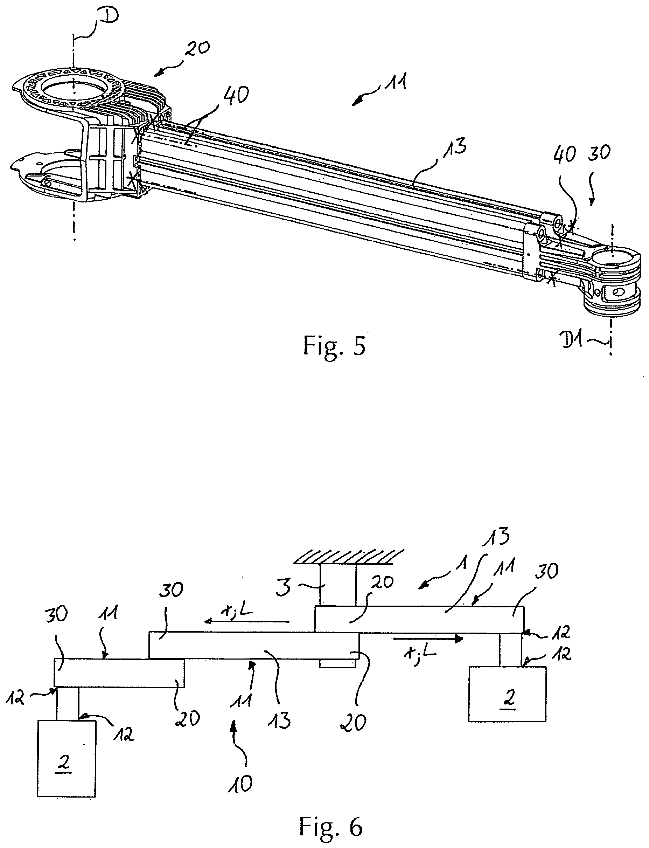

In der Figur 1B ist die Buchse 20 von einer inneren Seite gezeigt, von welcher Befestigungsmittel (nicht dargestellt) montierbar sind. Der Montageabschnitt 25 weist vier Durchführungen 25.5 für Befestigungsmittel auf, insbesondere für Schrauben. An jeder Durchführung 25.5 ist eine Anlagefläche 25.5a für Befestigungsmittel ausgebildet. Der Montageabschnitt 25 weist ferner eine Aussparung 25.7 für Leitungen oder Kabel auf, welche zwischen zwei Durchführungen 25.5 für Befestigungsmittel angeordnet ist. Der Tragarm 13 ist also über wenigstens ein, vorzugsweise vier Befestigungsmittel 40, die z. B. als Schrauben ausgebildet sind, an der Buchse 20 zu fixieren (vgl. Figur 5), die die Sicherung 25.1 an einer oder mehreren Ecken, insbesondere vier Ecken, flankieren, so dass eine sowohl drehfeste als auch in axialer Richtung (also entlang der Längsachse L) des Tragarms 13 bzw. in radialer Richtung der Buchse 20 zugfeste Verbindung zwischen dem Tragarm 13 und der Buchse 20 erreicht wird.

In der Figur 1C ist erkennbar, dass die Sicherung 25.1 den unteren Abschnitt der Aussparung 25.7 bildet. An einer Unterseite des oberen Ringabschnitts 22a sind Gewindebohrungen 22.6 zur Befestigung von Mitteln zur Kabelführung vorgesehen.

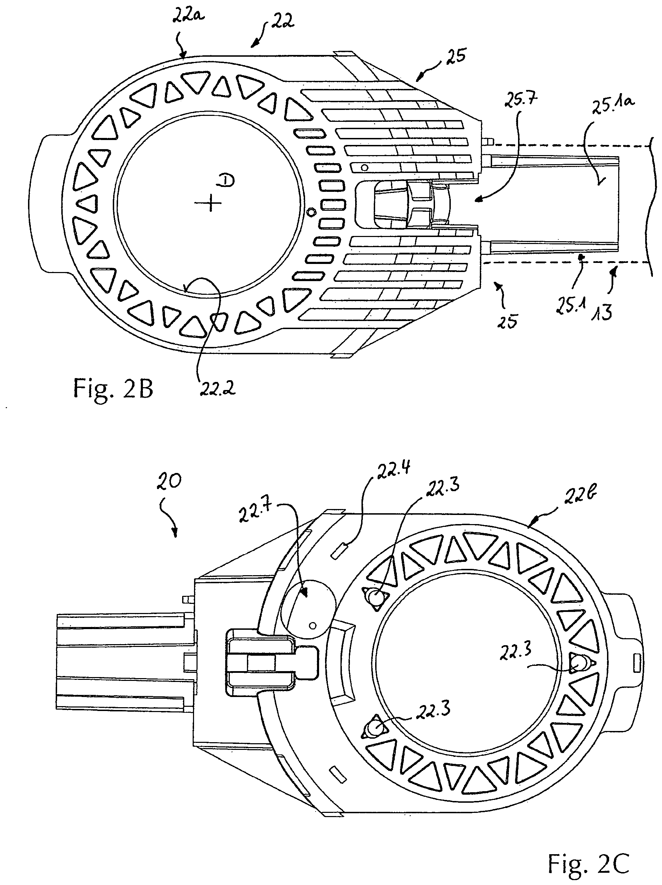

In den Figuren 2A, 2B, 2C ist der Aufbau der Buchse 20 in weiteren Ansichten gezeigt.

In der Figur 2A ist die Buchse 20 in einer Ausrichtung gezeigt, bei welcher die radiale Richtung r der horizontalen Richtung entspricht. In dieser Ausrichtung wird die Buchse 20 bevorzugt montiert, so dass sich die Buchse 20 um eine vertikal ausgerichtete Drehachse drehen kann. Die von den Ringabschnitten 22a, 22b definierte Drehachse D ist exakt in vertikaler Richtung z ausgerichtet. Die Stirnseite 25a erstreckt sich in einer Ebene orthogonal zur radialen Richtung r. Die Sicherung 25.1 erstreckt sich in radialer Richtung r.

In der Figur 2B ist erkennbar, dass die Aussparung 25.7 nur im Montageabschnitt 25, nicht jedoch im Ringabschnitt 22 ausgebildet ist. Kabel oder Leitungen können vom Tragarm (nicht dargestellt) durch die Aussparung 25.7 zwischen dem oberen Ringabschnitt 22a und dem unteren Ringabschnitt in Richtung der Drehachse D geführt werden.

In der Figur 2C ist erkennbar, dass der Auflageabschnitt, die Auflagefläche oder die Auflagekontur 25.1a der Sicherung 25.1 durch eine in sich geschlossene Fläche gebildet ist, welche in Hinblick auf die Breite des durch die Strichlinie angedeuteten Tragarms 13 möglichst maximal groß ist. Die Breite des Auflageabschnitts 25.1a beträgt insbesondere mindestens die Hälfte, bevorzugt mindestens 3/5 der Breite des Tragarms. Hierdurch können größere Lasten ohne Spannungsspitzen oder ohne das Risiko der Verformung des Profils des Tragarms übertragen werden. An einer Unterseite des unteren Ringabschnitts 22b sind eine oder mehrere Aussparungen 22.7 für Leitungen oder Kabel vorgesehen. Mit anderen Worten können Kabel wahlweise zwischen den Ringabschnitten zur Spindel (nicht dargestellt) oder nach unten oder oben aus der Buchse 20 heraus geführt werden. Die Kabelführung ist ein Aspekt, welcher bei modulartig aufgebauten Tragsystemen berücksichtigt werden muss, da in den meisten Fällen die vom Tragsystem gehaltenen medizintechnischen Einrichtungen eine Mehrzahl von elektrischen oder sonstigen Zufuhrleitungen erfordern. Die Buchse 20 ermöglicht unterschiedliche Anordnungs-Varianten für diese Leitungen, und bleibt dabei auf einfache Weise montierbar.

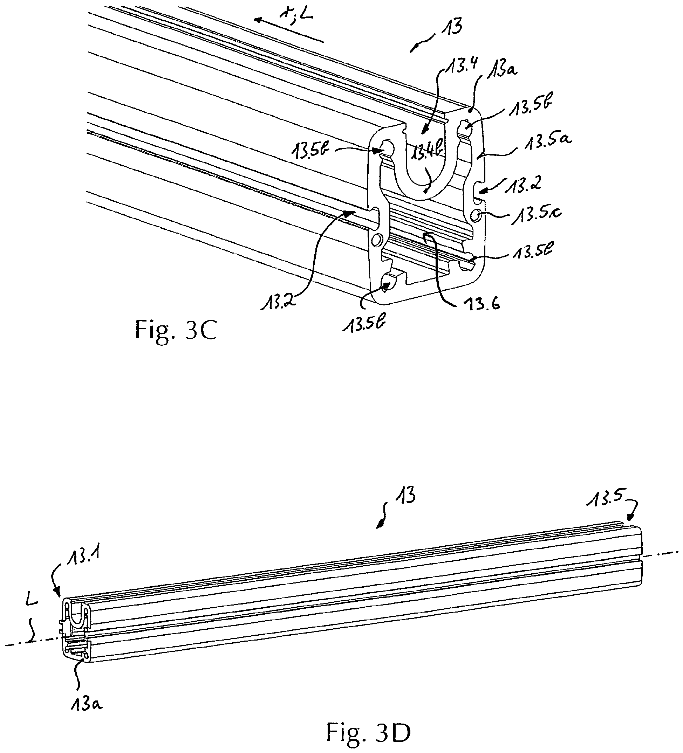

In den Figuren 3A, 3B, 3C ist das Querschnittsprofil Q eines sich entlang einer Längsachse L erstreckenden Tragarms 13 gezeigt. In einem an der zuvor beschriebenen Buchse 20 montierten Zustand ist die Längsachse L in radialer Richtung r ausgerichtet. Der Tragarm 13 ist durch eine umlaufende Wandung 13a gebildet, welche wenigstens drei Funktionen erfüllt, insbesondere ein Befestigen, ein Sichern und/oder Zentrieren sowie ein Anordnen von Kabeln.

In der Figur 3A ist eine erste Montageschnittstelle 13.1 gezeigt, wobei die Montageschnittstelle 13.1 im Wesentlichen durch eine Stirnseite oder Stirnfläche 13.1a des Tragarms 13 gebildet ist. An der Montageschnittstelle 13.1 sind vier Aufnahmen 13.1b ausgebildet, insbesondere in Form von Schraubkanälen für Befestigungsmittel. Dabei sind die Aufnahmen 13.1b in der Wandung 13a ausgebildet.

An der Montageschnittstelle 13.1 sind Zapfen 13.1c zur Verdrehsicherung und zur vertikalen Positionierung sowie Ausfräsungen 13.1d zur horizontalen Positionierung ausgebildet.

Die Wandung 13a weist vier Ecken oder Faltungen 13a.1 auf, in welchen die Aufnahmen 13.1b angeordnet sind.

Die Wandung 13a bildet ferner einen Kanal 13.4, insbesondere einen nach oben offenen Kabelkanal. In einem oberen Abschnitt des Kanals 13.4 sind zwei Nuten 13.4a zum Einrasten von Kabelsicherungsmitteln ausgebildet.

Die Wandung 13a weist einen Lagerungsabschnitt 13.4b auf, welcher eingerichtet ist, auf/an der geometrisch korrespondierenden Sicherung 25.1 gelagert zu werden.

In der Figur 3B ist eine am Lagerungsabschnitt 13.4b ausgebildete Lagerfläche 13.4c erkennbar, auf welcher der Tragarm 13 an der Sicherung 25.1 lagerbar ist. Die Lagerfläche 13.4c und die Auflagefläche 25.1a der Sicherung 25.1 weisen bevorzugt zumindest annähernd denselben Krümmungsradius auf. Aus der Figur 3B geht hervor, dass der Lagerungsabschnitt 13.4b ein U-Profil bildet. Hierdurch kann eine große Gewichtskraft aufgenommen werden. Das U-Profil kann eine hohe Tragkraft und Formbeständigkeit sicherstellen. Dank des U-Profils kann die Sicherung 25.1 in radialer Richtung vergleichsweise kurz ausgebildet sein. An einer Außenseite der Wandung 13a ist eine Nut 13.2 zum Befestigen von Verkleidungsteilen vorgesehen.

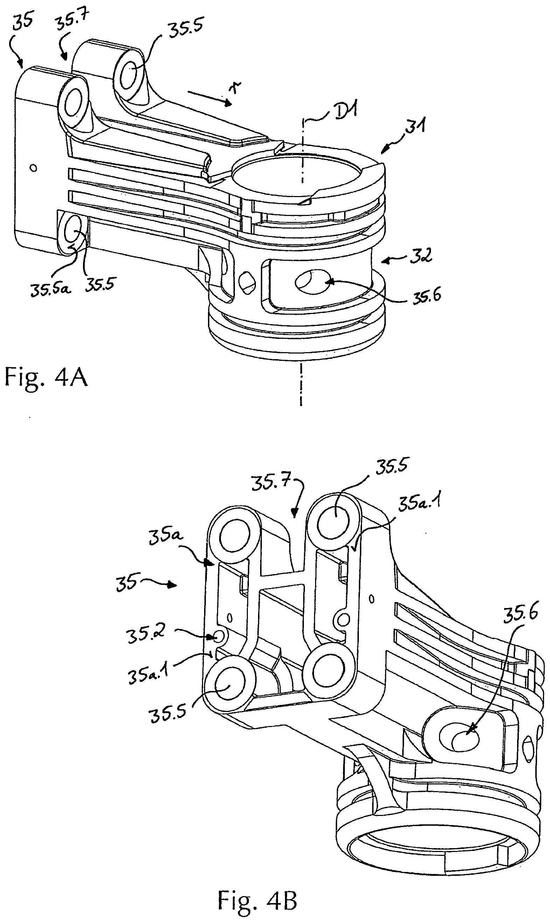

In der Figur 3C ist eine zweite Montageschnittstelle 13.5 des Tragarms 13 gezeigt. Die Montageschnittstellen 13.1, 13.5 sind jeweils an einem freien Ende des Tragarms 13 vorgesehen, speziell an der jeweiligen Stirnseite 13.1a, 13.5a. Die Montageschnittstelle 13.5 umfasst vier Aufnahmen 13.5b für Befestigungsmittel, insbesondere in Form von Schraubkanälen. Bevorzugt sind die Aufnahmen an beiden Montageschnittstellen identisch ausgebildet. An der Stirnseite 13.5a sind ferner sich in radialer Richtung r bzw. in Längsrichtung L erstreckende Stiftbohrungen 13.5c zur Verdrehsicherung und zur vertikalen Positionierung vorgesehen. In diese Stiftbohrungen 13.5c können Stifte zum Ausrichten und Sichern einer weiteren Buchse (vgl. Fig. 4A, 4B) eingebracht werden.

In der Figur 3D ist der Tragarm 13 in seiner Gesamtheit dargestellt. Die Montageschnittstellen 13.1, 13.5 sind jeweils an einem freien Ende des Tragarms 13 vorgesehen. Es ist erkennbar, dass der Tragarm 13 in Form eines sich geradlinig entlang der Längsachse L erstreckenden Strangpressprofils ausgebildet ist.

In den Figuren 4A, 4B ist eine weitere Buchse 30 gezeigt, welche eine weitere Drehachse D1 definiert. Die weitere Buchse 30 wird bevorzugt an einem vorderen, zu einer medizintechnischen Einrichtung weisenden Ende des Tragarms montiert. Wie auch die erste (hintere) Buchse 20 weist die weitere Buchse 30 einen Kupplungsteil 31 mit Ringabschnitt 32 sowie einen Montageabschnitt 35 auf. Der Montageabschnitt 35 weist eine Stirnseite 35a mit einer oder mehreren Stirnflächen 35a.1 sowie vier Durchführungen 35.5 für Befestigungsmittel auf, wobei die Durchführungen jeweils eine Anlagefläche 35.5a zur Anlage des Befestigungsmittels aufweisen, z.B. zur Anlage eines Schraubenkopfes. Der Montageabschnitt 35 weist ferner einen formschlüssigen Sicherungsabschnitt 35.2 gegen Vertikalbewegung und/oder Verdrehen auf, hier in Form von zwei Stiftbohrungen.

Am Kupplungsteil 31 sind Durchgangslöcher 35.6 fluchtend zu zwei der Durchführungen eingebracht. Die Durchgangslöcher 35.6 erleichtern die Montage von radial außen. Zwischen zwei der Durchgangslöcher 35.6, nämlich den oberen Durchgangslöchern 35.6, ist eine Aussparung 35.7 für Leitungen oder Kabel vorgesehen.

In der Figur 5 ist ein drehlagerbarer Ausleger 11 umfassend die hintere Buchse 20 und die vordere Buchse 30 sowie den Tragarm 13 gezeigt. Ferner sind Befestigungsmittel 40 in Form von Schrauben angedeutet.

In der Figur 6 ist eine an einer Decke montierte Stativvorrichtung 1 gezeigt, welche drei Ausleger 11 umfasst, die jeweils eine hintere Buchse 20, einen Tragarm 13 und eine vordere Buchse 30 umfassen. Zwei der Tragarme 13 lagern drehbar an einer Spindel 3. Die Stativvorrichtung 1 positioniert und hält zwei medizintechnische Einrichtungen 2, welche in Drehgelenken 12 am jeweiligen Tragarm 13 bzw. der jeweiligen vorderen Buchse 30 gelagert sind.

### Bezugszeichenliste

- 1: Stativvorrichtung, insbesondere Deckenstativvorrichtung
- 2: medizintechnische Einrichtung, insbesondere Versorgungskonsole
- 3: Verbindungsbauteil, insbesondere Spindel

- 10: Tragsystem
- 11: drehlagerbarer Ausleger, umfassend zwei Buchsen und einen Tragarm
- 12: Drehgelenk
- 13: Tragarm (Federarm)
- 13a: umlaufende Wandung
- 13a.1: Ecke oder Faltung
- 13.1: erste Montageschnittstelle, insbesondere erste Stirnseite des Tragarms
- 13.1a: erste Stirnfläche des Tragarms
- 13.1b: Aufnahme, insbesondere Schraubkanal für Befestigungsmittel
- 13.1c: Zapfen zur Verdrehsicherung und zur vertikalen Positionierung
- 13.1d: Ausfräsung zur horizontalen Positionierung
- 13.2: Nut zum Befestigen von Verkleidungsteilen
- 13.4: Kanal, insbesondere nach oben offener Kabelkanal
- 13.4a: Nut zum Einrasten von Kabelsicherungsmitteln
- 13.4b: Lagerungsabschnitt
- 13.4c: Lagerfläche am Lagerungsabschnitt
- 13.5: zweite Montageschnittstelle, insbesondere zweite Stirnseite des Tragarms
- 13.5a: zweite Stirnfläche des Tragarms
- 13.5b: Aufnahme für Befestigungsmittel an zweiter Montageschnittstelle
- 13.5c: Stiftbohrungen zur Verdrehsicherung und zur vertikalen Positionierung
- 13.6: Innenraum des Tragarms

- 20: (erste, hintere) Buchse eingerichtet zum Kuppeln an das Verbindungsbauteil
- 21: Kupplungsteil
- 22: Ringabschnitt einer gabelförmigen Buchse
- 22a: oberer Ringabschnitt
- 22b: unterer Ringabschnitt
- 22.1: Stirnfläche
- 22.2: nach innen weisende Mantelfläche (Lagersitz)
- 22.3: Ausnehmung zum Einrasten eines Schleifrings
- 22.4: Ausnehmung, insbesondere Durchgangsbohrung, zum Einrasten einer Abdeckkappe
- 22.5: Gewindebohrung für Bremsschraube
- 22.6: Gewindebohrung zur Befestigung von Mitteln zur Kabelführung
- 22.7: Aussparung für Leitungen oder Kabel
- 23: Aufnahme für Riegel zum Einstellen einer Anschlagsposition bzw. Drehposition
- 25: Montageabschnitt
- 25a: Stirnseite
- 25a.1: Stirnfläche
- 25.1: Sicherung für Tragarm
- 25.1a: Auflageabschnitt, Auflagefläche oder Auflagekontur
- 25.2: formschlüssiger Sicherungsabschnitt gegen Vertikalbewegung und/oder Verdrehen
- 25.3: formschlüssiger Sicherungsabschnitt gegen Axialbewegung und/oder Verdrehen
- 25.4: Schrauböse zum Anschrauben einer Verkleidung
- 25.5: Durchführung für Befestigungsmittel, insbesondere für Schraube
- 25.5a: Anlagefläche für Befestigungsmittel
- 25.7: Aussparung für Leitungen oder Kabel

- 30: weitere (vordere) Buchse, am freien Ende des Tragarms angeordnet
- 31: Kupplungsteil
- 32: Ringabschnitt
- 35: Montageabschnitt
- 35a: Stirnseite
- 35a.1: Stirnfläche
- 35.2: formschlüssiger Sicherungsabschnitt gegen Vertikalbewegung und/oder Verdrehen
- 35.5: Durchführung für Befestigungsmittel, insbesondere für Schraube
- 35.5a: Anlagefläche für Befestigungsmittel
- 35.6: Durchgangsloch fluchtend zur Durchführung
- 35.7: Aussparung für Leitungen oder Kabel

- 40: Befestigungsmittel, insbesondere selbstfurchende Schraube

- D: Drehachse, insbesondere vertikal ausgerichtete Hochachse
- D1: von der weiteren Buchse definierte Drehachse
- L: Längsachse des Tragarms
- Q: Querschnittsprofil des Tragarms
- r: radiale Richtung
- z: vertikale Richtung

## Patentansprüche

1. Stativvorrichtung (1) zur Anordnung im Operationssaal und zum Positionieren/Halten einer medizintechnischen Einrichtung (2) im Operationsaal, umfassend wenigstens eine Buchse (20) für eine Stativvorrichtung (1) zur Anordnung im Operationssaal, umfassend
- einen Kupplungsteil (21), mittels welchem die Buchse (20) um eine Drehachse (D) und/oder höhenverstellbar entlang der Drehachse an einem Verbindungsbauteil (3) der Stativvorrichtung (1) gelagert ist, wobei der Kupplungsteil im Wesentlichen durch zwei Ringabschnitte (22, 22a, 22b) gebildet ist, wodurch die Buchse gabelförmig ausgebildet ist;
- einen Abschnitt, an welchem die Buchse (20) mit einem Tragarm (13) der Stativvorrichtung (1) verbindbar ist;
wobei der Abschnitt ein Montageabschnitt (25) ist, mittels welches die Buchse (20) am Tragarm reversibel montiert ist, und
- wenigstens einen Tragarm (13),
wobei die Stativvorrichtung (1) ein Verbindungsbauteil (3) in Form einer Spindel aufweist, an welchem der Tragarm (13) mittels der Buchse reversibel montiert ist und **dadurch gekennzeichnet, dass**
der Montageabschnitt (25) wenigstens eine zur Drehachse (D) weisende Anlagefläche (25.5a) aufweist, an welcher ein Befestigungsmittel (40) zur Übertragung einer nach radial außen in radialer Richtung im Wesentlichen orthogonal zur Drehachse (D) wirkenden Normalkraft zur Anlage bringbar ist.

2. Stativvorrichtung (1) nach Anspruch 1, wobei die Buchse (20) durch ihren Montageabschnitt (25) am Tragarm mittels mehrerer Befestigungsmittel (40) reversibel montierbar ist.

3. Stativvorrichtung (1) nach Anspruch 1 oder 2, wobei der Montageabschnitt (25) eingerichtet ist, die Buchse (20) ohne Stoffschluss form- und/oder kraftschlüssig, bevorzugt zumindest im Wesentlichen kraftschlüssig, in einer vordefinierten Relativposition reversibel montierbar/demontierbar am Tragarm (13) zu befestigen.

4. Stativvorrichtung (1) nach einem der Ansprüche 1 bis 3, wobei der Montageabschnitt (25) wenigstens eine Durchführung (25.5) zur Anordnung eines Befestigungsmittels aufweist.

5. Stativvorrichtung (1) nach einem der vorhergehenden Ansprüche, wobei der Montageabschnitt (25) eine Sicherung (25.1) zum Abstützen des Tragarms aufweist, insbesondere eine Sicherung, eingerichtet zum Halten des Tragarms unabhängig von Befestigungsmitteln (40).

6. Stativvorrichtung (1) nach Anspruch 5, wobei die Sicherung (25.1) in radialer Richtung im Wesentlichen orthogonal zur Drehachse (D) hervorsteht, wobei die Sicherung (25.1) ein Querschnittsprofil aufweist, welches geometrisch korrespondierend zum Querschnittsprofil (Q) des Tragarms (13) ausgebildet ist.

7. Stativvorrichtung (1) nach Anspruch 5 oder 6, wobei die Sicherung (25.1) als Längsprofil ausgebildet ist, das geeignet ist, in einen Innenraum (13.6) des Tragarms (13) eingesteckt zu werden, und die Sicherung (25.1) eine Auflagekontur (25.1a) aufweist, die mit einer Kontur der Lagerfläche (13.4c) an einem Lagerabschnitt (13.4b) des Tragarms (13) korrespondiert, so dass im eingesteckten Zustand der Sicherung (25.1) in den Innenraum (13.6) des Tragarms (13) die Lagerfläche (13.4c) sich in Anlage an der korrespondierenden Auflagekontur (25.1a) der Sicherung (25.1) befindet.

8. Stativvorrichtung (1) nach einem der Ansprüche 5 bis 7, wobei die Sicherung (25.1) einen bevorzugt konkav nach innen und unten gewölbten Auflageabschnitt (25.1a) aufweist, welcher eingerichtet ist, den Tragarm zu lagern und in vertikaler Richtung abzustützen, wobei sich der Auflageabschnitt (25.1a) bevorzugt zumindest annähernd in radialer Richtung orthogonal zur Drehachse (D) erstreckt.

9. Stativvorrichtung (1) nach einem der vorhergehenden Ansprüche, wobei der Montageabschnitt (25) wenigstens einen formschlüssigen Sicherungsabschnitt (25.2, 25.3) gegen Vertikalbewegung und/oder Verdrehen oder gegen Axialbewegung und/oder Verdrehen aufweist.

10. Stativvorrichtung (1) nach einem der vorhergehenden Ansprüche, wobei die beiden Ringabschnitte (22, 22a, 22b) Lagersitze für ein Drehlager aufweisen.

11. Stativvorrichtung (1) nach einem der vorhergehenden Ansprüche, wobei die Buchse (20) ein Gussteil ist, insbesondere aus Aluminium oder einer Aluminiumlegierung.

12. Stativvorrichtung (1) nach einem der vorhergehenden Ansprüche, wobei zwei Buchsen und ein Tragarm jeweils einen bevorzugt drehlagerbaren Ausleger (11), bestehend aus wenigstens drei reversibel montierbaren Komponenten, bilden.

13. Stativvorrichtung (1) nach Anspruch 12, wobei die wenigstens zwei Buchsen an einer jeweiligen Montageschnittstelle (13.1, 13.5) am Tragarm montiert oder montierbar sind, wobei die Montageschnittstellen (13.1, 13.5) jeweils an einem freien Ende des Tragarms angeordnet sind, wobei die Montageschnittstellen (13.1, 13.5) bevorzugt identisch ausgebildet sind, wobei die Montageschnittstellen (13.1, 13.5) bevorzugt jeweils fluchtend zur anderen Montageschnittstelle (13.1, 13.5) angeordnete Aufnahmen für Befestigungsmittel aufweisen.

14. Stativvorrichtung (1) nach Anspruch 13, wobei der Montageabschnitt (25) und die Montageschnittstelle (13.1, 13.5) eingerichtet sind, die Buchse (20) und den Tragarm (13) ohne Stoffschluss form- und/oder kraftschlüssig in einer vordefinierten Relativposition reversibel montierbar/demontierbar aneinander zu befestigen,
wobei der Montageabschnitt (25) wenigstens eine Durchführung (25.5) zur Anordnung eines Befestigungsmittels aufweist, wobei der Montageabschnitt (25) wenigstens eine zur Drehachse (D) weisende Anlagefläche (25.5a) aufweist, an welcher ein Befestigungsmittel (40) zur Übertragung einer nach radial außen in radialer Richtung im Wesentlichen orthogonal zur Drehachse wirkenden Normalkraft zur Anlage bringbar ist, wobei der Montageabschnitt (25) eine nach radial außen weisende Stirnseite (25a) aufweist, welche geometrisch korrespondierend zu einer Stirnseite des Tragarms ausgebildet ist, und
wobei die Buchse (20) eine Sicherung (25.1) zum Halten des Tragarms aufweist.

15. Verwendung einer Stativvorrichtung (1) nach einem der vorhergehenden Ansprüche im Operationssaal zum Positionieren/Halten einer medizintechnischen Einrichtung (2),
wobei die Stativvorrichtung (1) ein Verbindungsbauteil (3) aufweist, an welchem der Tragarm (13) mittels der Buchse (20) reversibel montiert ist, wobei die Buchse (20) einen Montageabschnitt (25) aufweist, wobei der Tragarm eine Montageschnittstelle (13.1, 13.5) aufweist, und wobei der Montageabschnitt (25) und die Montageschnittstelle (13.1, 13.5) geometrisch korrespondierend zueinander ausgebildet sind.

## Claims

1. Stand apparatus (1) for arrangement in an operating theater and for positioning/holding a medical technology device (2) in an operating theater, comprising at least one bushing (20) for a stand apparatus (1) for arrangement in an operating theater, comprising
- a coupling part (21) by means of which the bushing (20) can be mounted around a rotation axis (D) and/or so as to be height-adjustable along the rotation axis on a connection module (3) of the stand apparatus (1), wherein the coupling part is formed essentially by two annular segments (22, 22a, 22b) so as to form the bushing in a fork-like manner,
- a segment to which the bushing (20) can be connected with a support arm (13) of the stand apparatus (1),
wherein the segment is a mounting segment (25) by means of which the bushing (20) is reversibly mounted on the support arm, and
- at least one support arm (13),
wherein the stand apparatus (1) has a connection module (3) in the form of a spindle on which the support arm is reversibly mounted by means of the bushing (20) and
**characterized in that**
the mounting segment (25) has at least one placement surface (25.5a) facing toward the rotation axis (D), on which placement surface a fastening means (40) can be placed for the transfer of a normal force that acts radially outward in a radial direction, essentially orthogonally to the rotation axis (D).

2. Stand apparatus (1) according to claim 1, wherein the bushing (20) by its mounting segment (25) can be reversibly mounted on the support arm by means of more than one fastening means (40).

3. Stand apparatus (1) according to claim 1 or 2, wherein the mounting segment (25) is configured to fasten the bushing (20) on the support arm (13) in a positive and/or non-positive fit preferably, at least essentially in a non-positive fit, without material bond, so that said bushing (20) can be reversibly assembled/disassembled at a predefined relative position.

4. Stand apparatus (1) according to any of claims 1 to 3, wherein the mounting segment (25) has at least one lead-through (25.5) for arrangement of a fastening means.

5. Stand apparatus (1) according to one of the preceding claims, wherein the mounting segment (25) has a safety device (25.1) to hold up the support arm - in particular, a safety device configured to hold the support arm independently of additional fastening means (40).

6. Stand apparatus (1) according to claim 5, wherein the safety device (25.1) protrudes in a radial direction essentially orthogonal to the rotational axis (D), wherein the safety device (25.1) has a cross-section profile which is designed to geometrically correspond to the cross-section profile (Q) of the support arm (13).

7. Stand apparatus (1) according to claim 5 or 6, wherein the safety device (25.1) is designed as a longitudinal profile that is suitable for insertion into an interior (13.6) of the support arm (13), and the safety device (25.1) has a support contour (25.1a) that corresponds to a contour of the bearing surface (13.4c) at a bearing segment (13.4b) of the support arm (13), such that - in the state in which the safety device (25.1) is inserted into the interior (13.6) of the support arm (13) - the bearing surface (13.4c) is placed on the corresponding support contour (25.1a) of the safety device (25.1).

8. Stand apparatus (1) according to one of claims 5 to 7, wherein the safety device (25.1) has a preferably concave support segment (25.1a) curved inward and downward, which support segment (25.1a) is configured to bear the support arm and support it in the vertical direction, wherein the support segment (25.1a) preferably extends at least approximately in the radial direction, orthogonal to the rotation axis (D).

9. Stand apparatus (1) according to any of the preceding claims, wherein the mounting segment (25) has at least one positive-fitting safety segment (25.2, 25.3) to counter vertical movement and/or twisting, or to counter axial movement and/or twisting.

10. Stand apparatus (1) according to any of the preceding claims, wherein the two annular segments (22, 22a, 22b) have bearing seats for a pivot bearing.

11. Stand apparatus (1) according to any of the preceding claims, wherein the bushing (20) is a cast part - in particular, made of aluminum or an aluminum alloy.

12. Stand apparatus (1) according to any one of the preceding claims, wherein two bushings and one support arm respectively form a boom (11) that is preferably rotatably mounted, which boom (11) is composed of at least three reversibly mountable components.

13. Stand apparatus (1) according to claim 12, wherein the at least two bushings are or can be mounted at a respective mounting interface (13.1, 13.5) on the support arm, wherein the mounting interfaces (13.1, 13.5) are respectively arranged at a free end of the support arm, wherein the mounting interfaces (13.1, 13.5) are preferably of identical design, wherein the mounting interfaces (13.1, 13.5) preferably have respective receptacles for fastening means, which receptacles are flush with the other mounting interface (13.1, 13.5).

14. Stand apparatus (1) according to claim 13, wherein the mounting segment (25) and the mounting interface (13.1, 13.5) are configured to fasten the bushing (20) and the support arm (13) to one another in a positive and/or non-positive fit, without material bonding, in a predefined relative position so that they can be reversibly assembled/disassembled,
wherein the mounting segment (25) has at least one lead-through (25.5) for arrangement of a fastening means, wherein the mounting segment (25) has at least one placement surface (25.5a) facing towards the rotation axis (D), on which placement surface (25.5a) a fastening means (40) for transfer of a normal force acting radially outwardly in a radial direction essentially orthogonal to the rotation axis can be placed, wherein the mounting segment (25) has a radially outwardly directed face (25a) which is designed to geometrically correspond to a face of the support arm, and
wherein the bushing (20) has a safety device (25.1) to hold the support arm.

15. Use of a stand apparatus (1) according to any of the preceding claims in an operating theater for positioning/holding a medical technology device (2),
wherein the stand apparatus (1) has a connection module (3) on which the support arm (13) is reversibly mounted by means of the bushing (20), wherein the bushing (20) has a mounting segment (25), wherein the support arm has a mounting interface (13.1, 13.5), and wherein the mounting segment (25) and the mounting interface (13.1, 13.5) are designed to geometrically correspond to one another.

## Revendications

1. Dispositif à trépied (1) destiné à un agencement dans un bloc opératoire et destiné à un positionnement/maintien d'un équipement médical (2) dans un bloc opératoire, comprenant au moins une douille (20) pour un dispositif à trépied (1) destiné à un agencement dans un bloc opératoire, comprenant
- une partie de couplage (21), au moyen de laquelle la douille (20) est montée autour d'un axe de rotation (D) et/ou de manière réglable en hauteur le long de l'axe de rotation au niveau d'un composant de liaison (3) du dispositif à trépied (1), dans lequel la partie de couplage est pour l'essentiel formée par le biais de deux sections annulaires (22, 22a, 22b), moyennant quoi la douille est réalisée en forme de fourche ;
- une section au niveau de laquelle la douille (20) peut être reliée à un bras de support (13) du dispositif à trépied (1) ;
dans lequel la section est une section de montage (25) au moyen de laquelle la douille (20) est montée de manière réversible sur le bras de support, et
- au moins un bras de support (13),
dans lequel le dispositif à trépied (1) présente un composant de liaison (3) en forme de broche au niveau duquel le bras de support (13) est monté de manière réversible au moyen de la douille et **caractérisé en ce que** la section de montage (25) présente au moins une surface d'appui (25.5a) orientée vers l'axe de rotation (D), surface d'appui au niveau de laquelle un moyen de fixation (40) est mis en appui pour le transfert d'une force verticale agissant radialement vers l'extérieur dans la direction radiale pour l'essentiel de manière orthogonale à l'axe de rotation (D).

2. Dispositif à trépied (1) selon la revendication 1, dans lequel la douille (20) peut être montée de manière réversible par le biais de sa section de montage (25) au niveau du bras de support au moyen de plusieurs moyens de fixation (40).

3. Dispositif à trépied (1) selon la revendication 1 ou 2, dans lequel la section de montage (25) est conçue pour fixer la douille (20) sans complémentarité de matières et/ou de forces, de préférence au moins pour l'essentiel par complémentarité de forces, au niveau du bras de support (13) réversible de manière à pouvoir être montée/démontée dans une position relative prédéfinie.

4. Dispositif à trépied (1) selon l'une quelconque des revendications 1 à 3, dans lequel la section de montage (25) présente au moins un passage (25.5) destiné à un agencement d'un moyen de fixation.

5. Dispositif à trépied (1) selon l'une quelconque des revendications précédentes, dans lequel la section de montage (25) présente une protection (25.1) destinée à un soutien du bras de support, en particulier une protection, conçue pour le maintien du bras de support indépendamment de moyens de fixation (40).

6. Dispositif à trépied (1) selon la revendication 5, dans lequel la protection (25.1) fait saillie dans la direction radiale pour l'essentiel de manière orthogonale à l'axe de rotation (D), dans lequel la protection (25.1) présente un profil de section transversale qui est réalisé pour correspondre de manière géométrique au profil de section transversale (Q) du bras de support (13).

7. Dispositif à trépied (1) selon la revendication 5 ou 6, dans lequel la protection (25.1) est réalisée en tant que profil longitudinal qui est adéquat pour être introduit dans un espace interne (13.6) du bras de support (13), et la protection (25.1) présente un contour de contact (25.1a) qui correspond à un contour de la surface porteuse (13.4c) au niveau d'une section porteuse (13.4b) du bras de support (13), de sorte que dans un état introduit de la protection (25.1) dans l'espace interne (13.6) du bras de support (13) la surface porteuse (13.4c) se trouve en appui au niveau du contour de contact correspondant (25.1a) de la protection (25.1).

8. Dispositif à trépied (1) selon l'une quelconque des revendications 5 à 7, dans lequel la protection (25.1) présente une section de contact (25.1a) de préférence concave vers l'intérieur et voûtée sur le dessous qui est conçue pour entreposer le bras de support et le soutenir dans la direction verticale, dans lequel la section de contact (25.1a) s'étend de préférence au moins approximativement dans la direction radiale de manière orthogonale à l'axe de rotation (D).

9. Dispositif à trépied (1) selon l'une quelconque des revendications précédentes, dans lequel la section de montage (25) présente au moins une section de protection (25.2, 25.3) à complémentarité de formes contre un mouvement vertical et/ou une déformation ou contre un mouvement axial et/ou une déformation.

10. Dispositif à trépied (1) selon l'une quelconque des revendications précédentes, dans lequel les deux sections annulaires (22, 22a, 22b) présentent des sièges de palier pour un palier pivotant.

11. Dispositif à trépied (1) selon l'une quelconque des revendications précédentes, dans lequel la douille (20) est une pièce moulée, en particulier à partir d'aluminium ou d'un alliage d'aluminium.

12. Dispositif à trépied (1) selon l'une quelconque des revendications précédentes, dans lequel deux douilles et un bras de support forment respectivement un avant-bras (11) pouvant de préférence pivoter, se composant d'au moins trois composants pouvant être montés de manière réversible.

13. Dispositif à trépied (1) selon la revendication 12, dans lequel les au moins deux douilles sont montées ou peuvent être montées au niveau du bras de support au niveau d'une interface de montage respective (13.1, 13.5), dans lequel les interfaces de montage (13.1, 13.5) sont respectivement agencées au niveau d'une extrémité libre du bras de support, dans lequel les interfaces de montage (13.1, 13.5) sont réalisées de préférence de manière identique, dans lequel les interfaces de montage (13.1, 13.5) présentent des logements, pour des moyens de fixation, disposés de préférence respectivement en alignement par rapport à d'autres interfaces de montage (13.1, 13.5).

14. Dispositif à trépied (1) selon la revendication 13, dans lequel la section de montage (25) et l'interface de montage (13.1, 13.5) sont conçues pour fixer la douille (20) et le bras de support (13) l'un à l'autre de manière à pouvoir être montés/démontés sans complémentarité de matières et/ou de forces dans une position relative prédéfinie,
dans lequel la section de montage (25) présente au moins un passage (25.5) destiné à un agencement d'un moyen de fixation, dans lequel la section de montage (25) présente au moins une surface d'appui (25.5a) orientée vers l'axe de rotation (D), au niveau de laquelle un moyen de fixation (40) peut être mis en appui radialement à l'extérieur dans la direction radiale pour l'essentiel de manière orthogonale à la force verticale agissant sur l'axe de rotation, dans lequel la section de montage (25) présente une face avant (25a) tournée radialement vers l'extérieur, laquelle est réalisée pour correspondre géométriquement à une face avant du bras de support, et
dans lequel la douille (20) présente une protection (25.1) destinée à un maintien du bras de support.

15. Utilisation d'un dispositif à trépied (1) selon l'une quelconque des revendications précédentes dans un bloc opératoire destiné à un positionnement/maintien d'un équipement médical (2),
dans lequel le dispositif à trépied (1) présente un composant de liaison (3), au niveau duquel le bras de support (13) est monté de manière réversible au moyen de la douille (20), dans lequel la douille (20) présente une section de montage (25), dans lequel le bras de support présente une interface de montage (13.1, 13.5), et dans lequel la section de montage (25) et l'interface de montage (13.1, 13.5) sont réalisées de manière à correspondre géométriquement l'une à l'autre.
